# EUROPEAN PATENT APPLICATION

(11) **EP 4 458 839 A1**
(43) Date of publication of application: **06.11.2024**
(21) Application number: 22916642.6
(22) Date of filing: 26.12.2022
(51) Int. Cl.: C07K 7/06, A61K 47/42

(54) **NOVEL CELL-PERMEABLE PEPTIDE AND USE THEREOF**

(30) Priority: 27.12.2021 KR 20210188180; 23.12.2022 KR 20220183261
(71) Applicant: Ajou University Industry-Academic Cooperation Foundation, Suwon-si, Gyeonggi-do 16499 (KR)
(72) Inventor: SOHN, Seonghyang, Suwon-si Gyeonggi-do 16699 (KR)
(74) Representative: Mewburn Ellis LLP
(86) International application number: PCT/KR2022/021269
(87) International publication number: WO 2023/128501

(57) **Abstract**

The present invention pertains to a novel cell-permeable peptide and a use thereof. A peptide having the amino acid sequence set forth in SEQ ID NO: 1 was found to exhibit cell permeability, maintain the cell permeability even when one amino acid sequence of the peptide is substituted with another amino acid, and be able to penetrate plasma membranes even when a non-cell-permeable peptide is connected to the N-terminus, to the C-terminus, or between the amino acids of the peptide, and thus can be advantageously used for a drug delivery composition.

## Description

### Technical Field

The present disclosure relates to a novel cell-permeable peptide and a use thereof.

### Background Art

Cells of higher animals have plasma membranes that separate their inside from their outside. The plasma membrane is essential for cytoprotection due to its role in preventing foreign substances such as viruses and bacteria from entering the cell, but hydrophobicity of a phospholipid bilayer that makes up the plasma membrane makes most peptides, proteins, nucleotides, and liposomes almost impossible to move into the cell, which, in turn, inhibits delivery of drugs for treatment in a pharmaceutical aspect.

Methods used to deliver drugs into the cell may be divided into a method of using an active transport system through ATP-binding transport molecules present in the plasma membrane and a method of forcibly penetrating the plasma membrane, and since very limited types of drugs are able to utilize the method with the active transport system involved, mainly used is the method of forcibly penetrating the plasma membrane. Specifically, drug delivery methods by means of cationic lipids/liposomes, cell-permeable peptides, micro-injections, and viruses are known, but due to limitations or side effects such as limitation of applicable cells and intracellular toxicity, research on drug delivery substances in an attempt to overcome the problems above is actively underway.

### Disclosure of the Invention

### Technical Goals

An object of the present disclosure is to provide a peptide with cell-permeability.

Another object of the present disclosure is to provide a peptide which includes a peptide without cell-permeability at the N-terminus or C-terminus, or between each amino acid of the peptide.

Another object of the present disclosure is to provide a nucleotide having base sequences encoding the peptide.

Another object of the present disclosure is to provide a vector including the nucleotide.

Another object of the present disclosure is to provide a transformant transformed by the vector.

Another object of the present disclosure is to provide a composition for drug delivery, including the peptide and a desired drug.

### Technical Solutions

To achieve the above object, the present disclosure provides a peptide represented by the following general sequence formula.

[General Formula] X1-X2-X3-X4-X5-X6-X7

The X1 is one selected from the group consisting of glycine, alanine, isoleucine, leucine, valine, asparagine, glutamine, arginine, lysine, aspartic acid, glutamic acid, tyrosine, tryptophan, phenylalanine, and proline,
the X2 is cysteine or arginine;
the X3 is cysteine or arginine;
the X4 is glycine or arginine;
the X5 is glutamic acid or arginine;
the X6 is isoleucine or arginine; and
the X7 is valine or arginine.

In addition, the present disclosure provides a peptide which includes a peptide without cell-permeability at the N-terminus or C-terminus, or between each amino acid of the peptide.

In addition, the present disclosure provides a nucleotide having a base sequence encoding the peptide.

In addition, the present disclosure provides a vector including the nucleotide.

In addition, the present disclosure provides a transformant transformed by the vector.

In addition, the present disclosure provides a composition for drug delivery, including the peptide and a desired drug.

### Advantageous Effects

According to the present disclosure, it was found that a peptide having amino acid sequences represented by SEQ ID NO: 1 exhibited cell permeability, maintained cell permeability even if one amino acid sequence of the peptide was substituted with another amino acid, and was capable of penetrating the plasma membrane even if a peptide without cell permeability was linked to N-terminus or C-terminus, or between each amino acid of the peptide, such that the peptide may be advantageously used as a composition for drug delivery.

### Brief Description of Drawings

FIG. 1 shows results of observing, via confocal microscopy, intracellular fluorescence expression of mouse-derived splenocytes that are cultured with treatment of fluorescent-labeled F1 and F2 peptides, respectively.
FIG. 2 shows results of observing, via confocal microscopy, intracellular fluorescence expression of mouse-derived peritoneal macrophages that are cultured with treatment of fluorescent-labeled F 1 and F2 peptides, respectively.
FIG. 3 shows results of observing, via confocal microscopy, fluorescence expression in tissues after applying fluorescent-labeled F1 and F2 peptides to normal skin and scratched skin of mice respectively, collecting tissues after 1~2 hours, and then performing cryosection.
FIG. 4 shows results of analyzing a frequency of intracellularly fluorescent-labeled cells via flow cytometry with mouse-derived splenocytes and lymph node cells cultured with treatment of fluorescent-labeled F1 and F2 peptides, respectively.

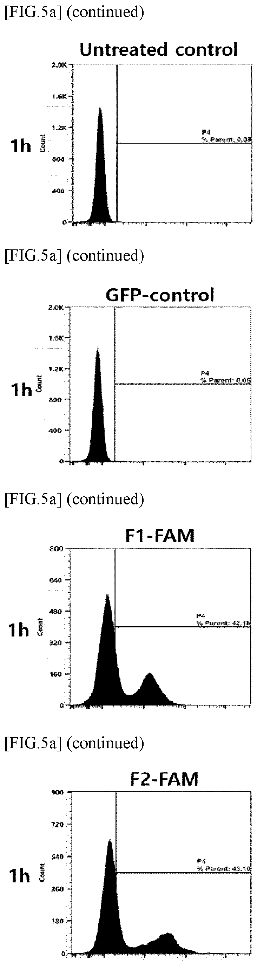

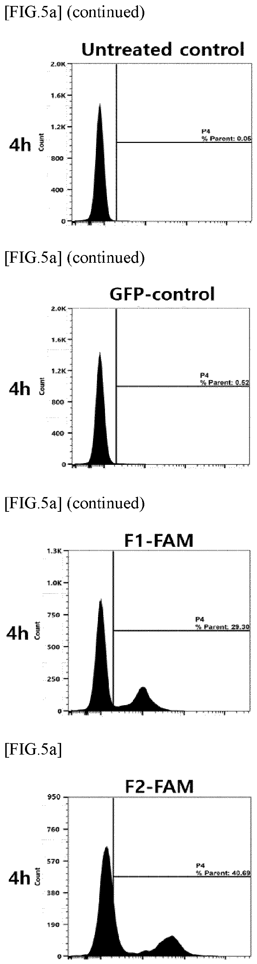

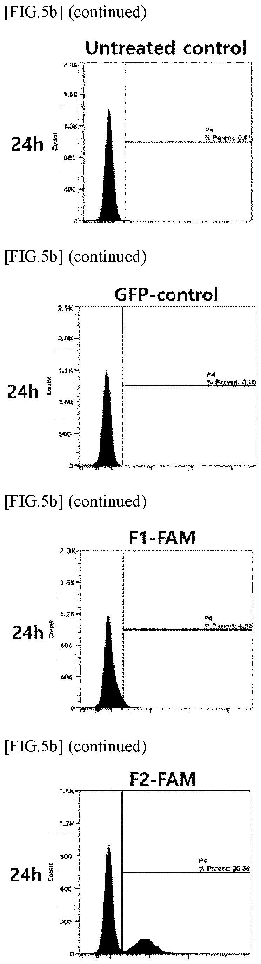

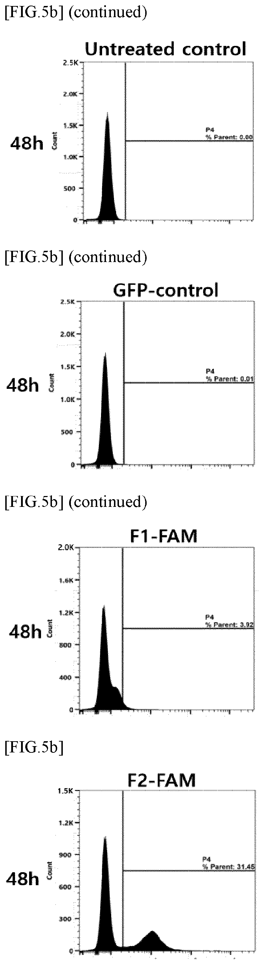

FIGS. 5A-5B are histogram data showing analysis results of A-D in FIG. 4.

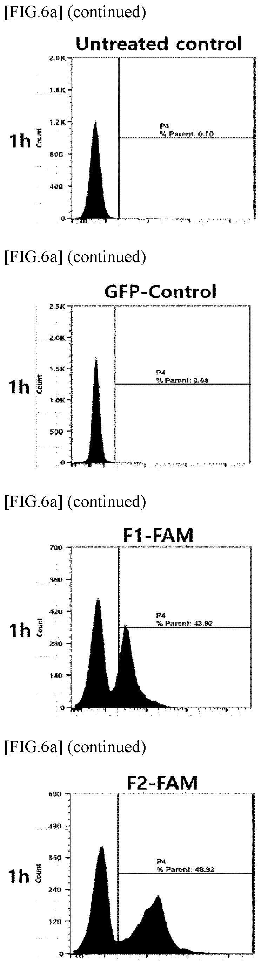

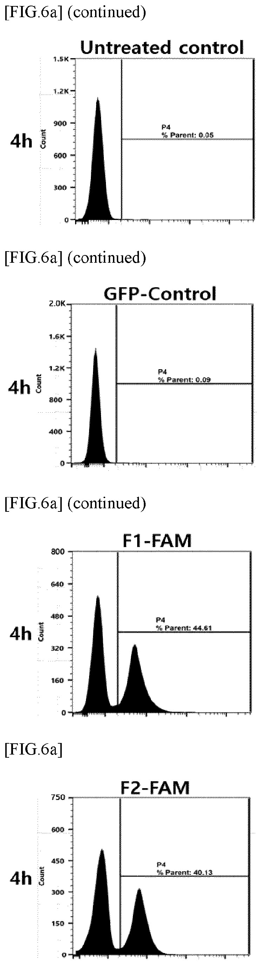

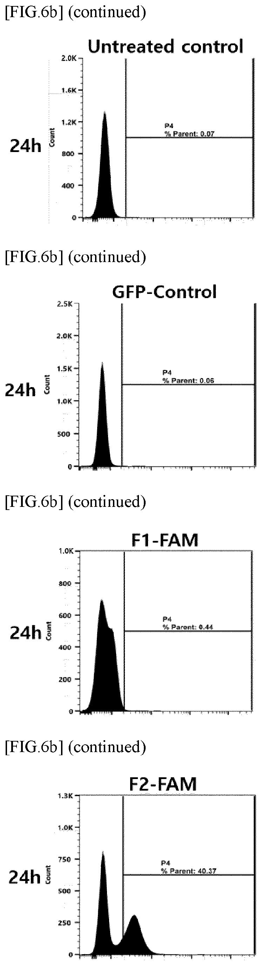

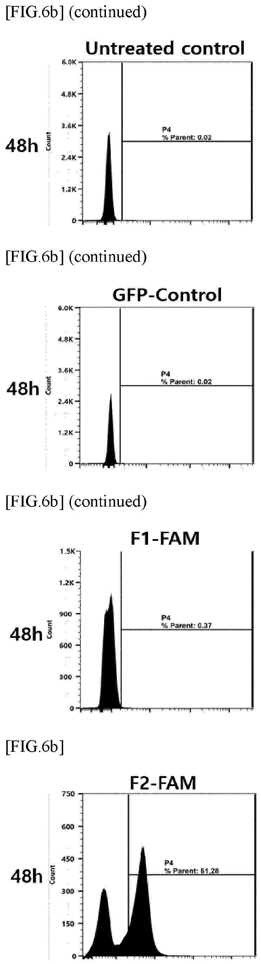

FIGS. 6A-6B are histogram data showing analysis results of E-H in FIG. 4.
FIG. 7 is a result of analyzing a frequency of intracellularly fluorescent-labeled cells over culture time through flow cytometry of mouse-derived splenocytes that are cultured with treatment of fluorescent-labeled peptides (SEQ ID NOS: 2 to 15), respectively.
FIGS. 8A-8B are histogram data showing analysis results at 72 hours of culture in FIG. 7.
FIG. 9 shows a result of analyzing a frequency of intracellularly fluorescent-labeled cells over culture time through flow cytometry of mouse-derived splenocytes that are cultured with treatment of fluorescent-labeled peptides (SEQ ID NOS: 16 to 21), respectively.
FIG. 10 is histogram data showing analysis results at 72 hours of culture in FIG. 9.
FIG. 11 shows a result of analyzing a frequency of intracellularly fluorescent-labeled cells at 24 hours of culture through flow cytometry of mouse-derived splenocytes that are cultured with treatment of fluorescent-labeled peptides (SEQ ID NOS: 16 to 21), respectively.
FIGS. 12A-12B are histogram data showing analysis results of FIG. 11.
FIG. 13 shows a result of analyzing a frequency of intracellularly fluorescent-labeled cells at 48 hours of culture through flow cytometry of mouse-derived splenocytes that are cultured with treatment of fluorescent-labeled peptides (SEQ ID NOS: 16 to 21), respectively.
FIGS. 14A-14B are histogram data showing analysis results of FIG. 13.
FIG. 15 shows results of observing intracellular fluorescence expression at low magnification through confocal microscopy of mouse-derived splenocytes that are cultured for 24 hours with treatment of fluorescent-labeled peptides (SEQ ID NOS: 16 to 21), respectively.
FIG. 16 shows results of observing intracellular fluorescence expression at high magnification through confocal microscopy of mouse-derived splenocytes that are cultured for 24 hours with treatment of fluorescent-labeled peptides (SEQ ID NOS: 16 to 21), respectively.
FIG. 17 shows results of observing, via confocal microscopy, fluorescence expression in tissues after applying fluorescent-labeled peptides (SEQ ID NOS: 16 to 21) to normal skin and scratched skin of mice respectively, collecting tissues after 4 hours, and then performing cryosection.

### Best Mode for Carrying Out the Invention

Hereinafter, the present disclosure will be described in detail.

The present disclosure provides a peptide represented by the following general sequence formula.

[General Formula] X1-X2-X3-X4-X5-X6-X7

The X1 may be one selected from the group consisting of glycine, alanine, isoleucine, leucine, valine, asparagine, glutamine, arginine, lysine, aspartic acid, glutamic acid, tyrosine, tryptophan, phenylalanine, and proline,
the X2 may be cysteine or arginine;
the X3 may be cysteine or arginine;
the X4 may be glycine or arginine;
the X5 may be glutamic acid or arginine;
the X6 may be isoleucine or arginine; and
the X7 may be valine or arginine.

The peptide may consist of any one amino acid sequence of SEQ ID NOS: 1 to 21.

In addition, the present disclosure provides a peptide which includes a peptide without cell permeability at the N-terminus or C-terminus, or between each amino acid of the peptide.

The peptide without cell permeability may consist of amino acids represented by SEQ ID NO: 31.

The peptide including a peptide without cell permeability may consist of, but is not limited to, any one of amino acid sequences represented by SEQ ID NOS: 22 to 30.

In addition, the present disclosure provides a nucleotide having base sequences encoding the peptide.

In addition, the present disclosure provides a vector including the nucleotide.

The vector may be, but is not limited to, one or more selected from the group consisting of plasmid vectors, cosmid vectors, bacteriophage vectors, and viral vectors. In addition, the vector of the present disclosure may be either a conventional cloning vector or an expression vector, wherein the expression vector includes a signal sequence or leader sequence for membrane targeting or secretion, in addition to expression regulatory sequences such as promoters, operators, initiation codons, termination codons, polyadenylating signals, and enhancers (promoting genes), and may be manufactured in a variety of ways depending on the purpose. In addition, the vector includes a selection marker for selecting a host cell including the vector and, if it is a replicable vector, it includes an origin of replication.

In addition, the present disclosure provides a transformant transformed by the vector.

The transformation may be performed by a transforming technique known to those of skilled in the art, preferably by one transforming technique selected from the group consisting of, but is not limited to, microprojectile bombardment, electroporation, calcium phosphate (CaPO₄) precipitation, calcium chloride (CaCl₂) precipitation, PEG-mediated fusion, microinjection, and liposome-mediated method.

The transformant may be, but is not limited to, one or more selected from the group consisting of Escherichia coli, Bacillus subtilis, Streptomyces, Pseudomonas, Proteus mirabilis, Staphylococcus, and Agrobacterium tumefaciens.

In addition, the present disclosure provides a composition for drug delivery, including the peptide and a desired drug.

The drug may be, but is not limited to, one selected from the group consisting of compounds, proteins, peptides, and nucleic acids.

In the composition, the peptide and the drug may form a covalent or non-covalent conjugate.

In addition, in the composition, the peptide and the drug may be covalently bonded to each other to form a conjugate.

The peptide may be labeled with one selected from the group consisting of, but is not limited to, chromogenic enzymes, radioisotopes, chromophores, luminescent materials, fluorescers, super paramagnetic particles, and ultrasuper paramagnetic particles.

### Modes for Carrying Out the Invention

Hereinafter, example embodiments will be described in detail to help understanding of the present disclosure. However, the following example embodiments are merely illustrative of the contents of the present disclosure, and the scope of the present disclosure is not limited to the following example embodiments. The example embodiments of the present disclosure are provided to more completely describe the present disclosure to those with ordinary skill in the art.

### [Experimental Example 1] Preparation of peptides

The fluorescent-labeled peptides shown in Table 1 below were prepared by requesting ANYGEN CO., LTD, and a peptide consisting of an amino acid sequence "GCCGEIV" among the peptides was named F 1 peptide. In addition, in order to determine whether the F1 peptide enables penetration of a peptide without cell permeability into a cell, a peptide, consisting of the amino acid sequence "GCDDDDDDDDCGEIV" prepared by inserting a peptide (DDDDDDDD; scramble D) consisting of 8 amino acids (aspartic acid) without cell permeability in the middle of the F1 peptide, was named F2 peptide.

**TABLE 1**

| Sequence Number | Peptide | Amino Acid Sequence |
|---|---|---|
| 1 | F1 | GCCGEIV |
| 2 | F1-A | ACCGEIV |
| 3 | F1-I | ICCGEIV |
| 4 | F1-L | LCCGEIV |
| 5 | F1-V | VCCGEIV |
| 6 | F1-N | NCCGEIV |
| 7 | F1-Q | QCCGEIV |
| 8 | F1-R | RCCGEIV |
| 9 | F1-K | KCCGEIV |
| 10 | F1-D | DCCGEIV |
| 11 | F1-E | ECCGEIV |
| 12 | F1-Y | YCCGEIV |
| 13 | F1-W | WCCGEIV |
| 14 | F1-F | FCCGEIV |
| 15 | F1-P | PCCGEIV |
| 16 | F1-R2 | GRCGEIV |
| 17 | F1-R3 | GCRGEIV |
| 18 | F1-R4 | GCCREIV |
| 19 | F1-R5 | GCCGRIV |
| 20 | F1-R6 | GCCGERV |
| 21 | F1-R7 | GCCGEIR |
| 22 | F1D2 | GDDDDDDDDCCGEIV |
| 23 | F2 | GCDDDDDDDDCGEIV |
| 24 | F1D4 | GCCDDDDDDDDGEIV |
| 25 | F1D5 | GCCGDDDDDDDDEIV |
| 26 | F1D6 | GCCGEDDDDDDDDIV |
| 27 | F1D7 | GCCGEIDDDDDDDDV |
| 28 | F1D8 | GCCGEIVDDDDDDDD |
| 29 | DF1 | DDDDDDDDGCCGEIV |
| 30 | DF1D | DDDDDDDDGCCGEIVDDDDDDDD |
| 31 | scramble D | DDDDDDDD |

### [Example 1] Analysis on cell permeability of F1 peptides

### 1-1. Analysis on splenocyte permeability by confocal microscopy

To determine the cell permeability of F1 peptides, 1 µg/mL of fluorescent-labeled F1 and F2 peptides were treated to cultures of splenocytes isolated from 4-week-old ICR mice, followed by culture for 24 hours. Afterwards, intracellular fluorescence expression over culture times (1, 4 and 24 hours) was observed by confocal microscopy. The untreated group with no peptide treatment involved was used as a control, alongside with a GFP peptide (HKFSVSGEGEGDATC) treated group without cell permeability and a scramble D treated group (negative control).

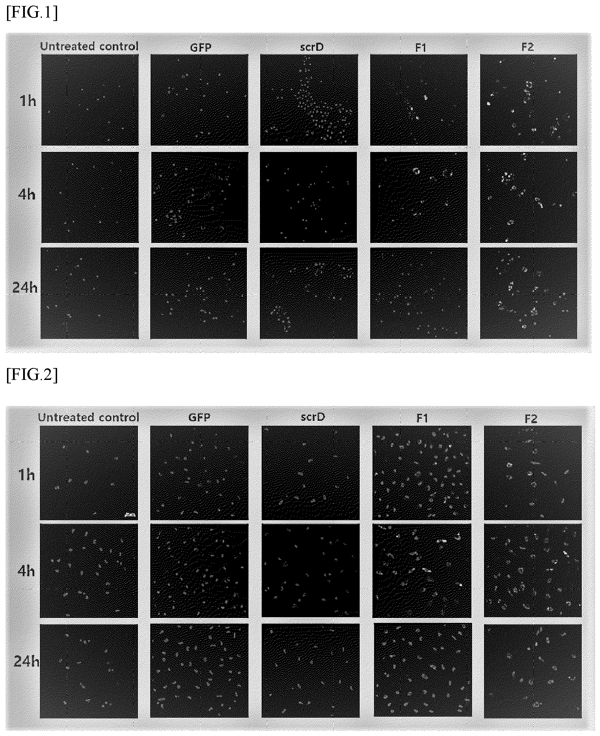

As a result, as shown in FIG. 1, it was found that both the F1 and F2 peptide treated groups showed fluorescence expression in splenocytes. From the above results, it was determined that the F1 peptide has the cell permeability for splenocytes and enables penetration of peptides without cell permeability into cells.

### 1-2. Analysis on peritoneal macrophage permeability by confocal microscopy

To determine the cell permeability of F1 peptides, 1 µg/mL of fluorescent-labeled F1 and F2 peptides was treated to cultures of peritoneal macrophages isolated from 4-week-old ICR mice, followed by culture for 24 hours. Afterwards, intracellular fluorescence expression over culture times (1, 4 and 24 hours) was observed by confocal microscopy.

As a result, as shown in FIG. 2, it was found that all the groups treated with both the F1 and F2 peptides showed fluorescence expression in peritoneal macrophages. From the above results, it was found that the F1 peptide has cell permeability for peritoneal macrophages and enables penetration of the peptide without cell permeability into the cell.

### 1-3. Analysis on skin tissue permeability by confocal microscopy

To identify the skin tissue permeability of the F1 peptide, the left leg of 4-week-old ICR mice was left unscratched, and the right leg was scratched with a needle. Afterwards, 1 µg/mL of fluorescent-labeled F1 and F2 peptides was applied to both legs with cotton swabs respectively, and after 1∼2 hours, skin (epidermis) tissues were collected, cryosectioned, and observed under confocal microscopy.

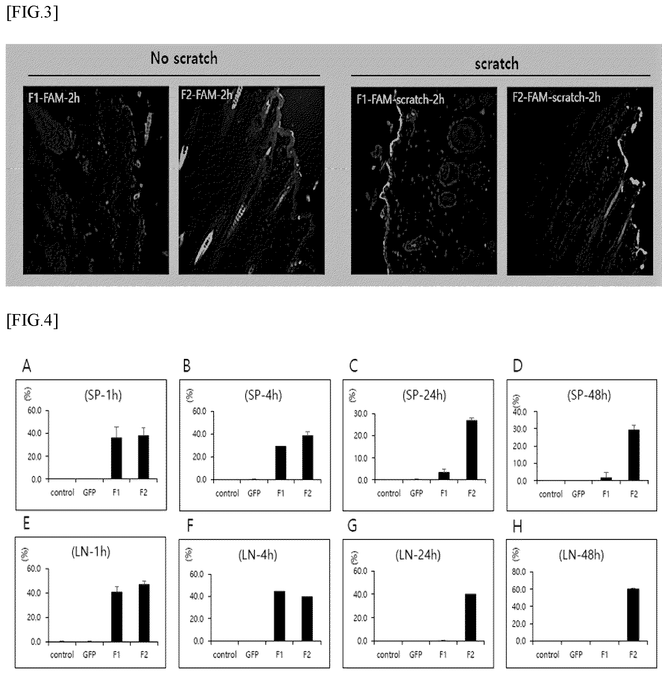

As a result, as shown in FIG. 3, it was found that both the F1 and F2 peptide treated groups showed fluorescence expression in tissues, and the F2 peptide showed strong fluorescence expression especially in hair follicles, with no significant difference observed in the fluorescence expression with or without scratches. From the above results, it was found that the F1 peptide has cell (tissue) permeability and enables penetration of the peptide without cell permeability into the cell (tissue).

### 1-4. Analysis on cell permeability via flow cytometry

To determine whether the F1 peptide has entered the cell by penetrating the plasma membrane, splenocytes and lymph node cells were isolated from 4-week-old ICR mice, cultured for 1 hour, treated with 1 µg/mL of fluorescent-labeled F1 and F2 peptides, respectively, and then cultured for 48 hours. Flow cytometry was then used to analyze a frequency of fluorescent-labeled cells over culture time (1, 4, 24, and 48 hours) to determine the frequency of cells into which the peptide moved.

As a result, as shown in FIGS. 4 (A-D) and 5A-5B, in the case of splenocytes, intracellular fluorescence expression was not observed in the control and the GFP peptide treated group, while about 30-40% of entire cells showed a positive cell frequency in F1 peptide over 4 hours and F2 peptide over 48 hours, identifying that intracellular fluorescence expression appeared. From the above results, it was found that the F1 peptide has cell permeability for splenocytes and enables penetration of peptides without cell permeability into the cells.

In addition, in the case of lymph node cells, as shown in FIGS. 4 (E-H) and 6A-6B, intracellular fluorescence expression was not observed in the untreated group (control) and GFP peptide treated groups, while about 40% of the entire cells for F1 peptide showed positive cell frequency over 4 hours, and about 60% of the entire cells for F2 peptide showed positive cell frequency over 48 hours. From the above results, it was found that the F1 peptide has the cell permeability for lymph node cells and enables penetration of the peptide without cell permeability into the cell.

### [Example 2] Analysis on cell permeability of modified F1 peptides

In the Example 1, it was found that the F1 peptide has the cell permeability and enables penetration of the peptide without cell permeability into the cell. Therefore, in order to determine whether some amino acid sequences of F1 peptide are modified while enabling penetration of peptides without cell permeability into the cells in a form other than the F2 peptide form, identified was the cell permeability of the peptide prepared by substituting one amino acid in the F1 peptide with another amino acid or inserting a sequence (DDDDDDDD, SEQ ID NO: 31) that does not penetrate the plasma membrane in the middle of the amino acid sequences of the F1 peptide like F2 peptide, as shown in Table 1 above.

### 2-1. Analysis on cell permeability of peptides with SEQ ID NOS: 2 to 21 via flow cytometry

In order to determine whether the cell permeability is derived if one amino acid of the F1 peptide is substituted by another amino acid, splenocytes were isolated from ICR 4-week-old mice and cultured for 1 hour, and then 1µg/mL of fluorescent-labeled peptide (SEQ ID NOS: 2 to 21) was treated to cells respectively, followed by culture for 72 hours. Afterwards, frequencies of fluorescent-labeled cells were analyzed over culture times (4, 24 and 48 and 72 hours) by flow cytometry to determine the frequency of cells into which the peptide moved.

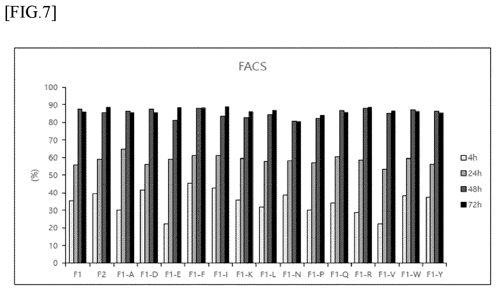

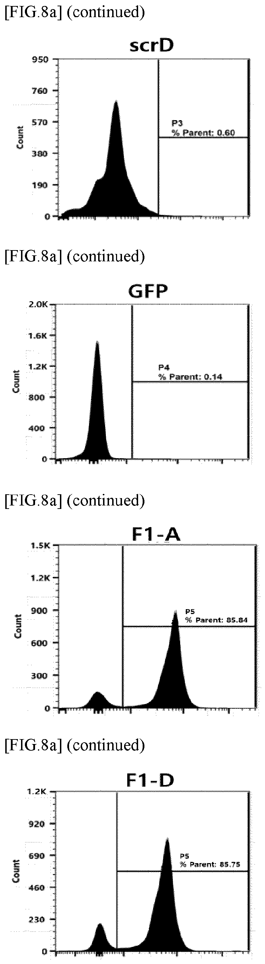

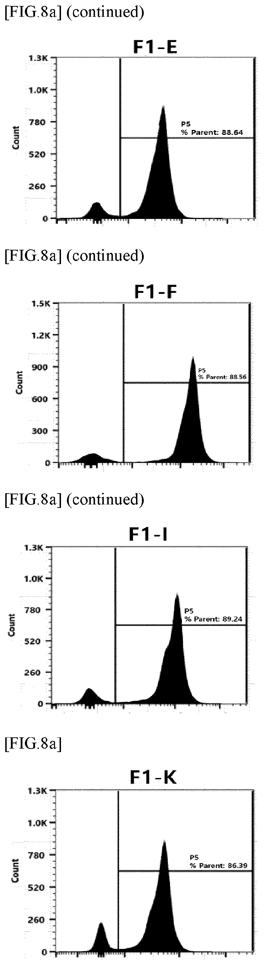

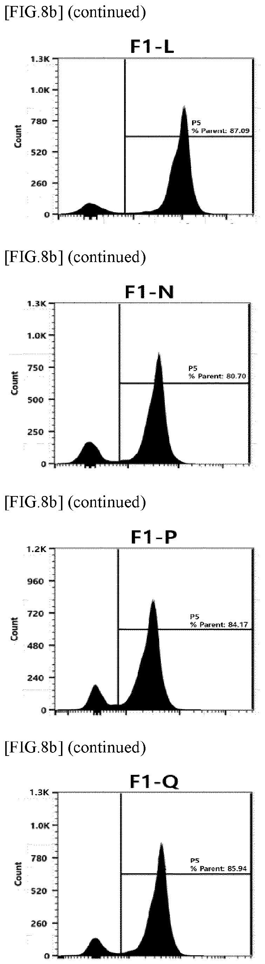

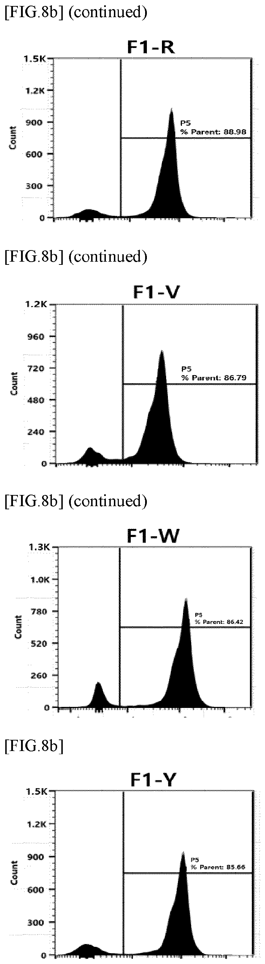

As a result, in the case of peptide (SEQ ID NOS: 2 to 15) in which a first amino acid sequence of the F1 peptide is substituted with another amino acid, as shown in FIGS. 7 and 8A-8B, both peptide treated groups showed similar positive cell frequencies, specifically, while showing positive cell frequencies of about 25-40% at 4 hours, more than 50% after 24 hours, and more than 80% after 48 hours, it was found that intracellular fluorescence expression increased over time, and high fluorescence expression rate was maintained up to 72 hours. From the above results, it was determined that a first amino acid of the F1 peptide retains its cell permeability even when it is substituted by another amino acid.

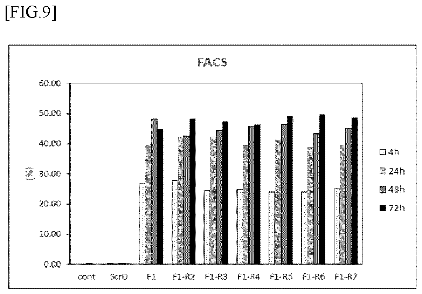

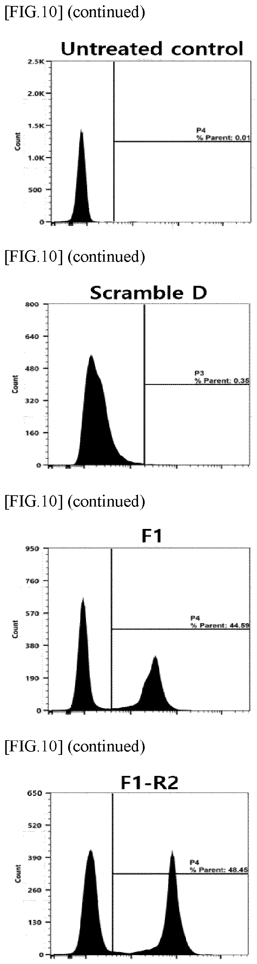

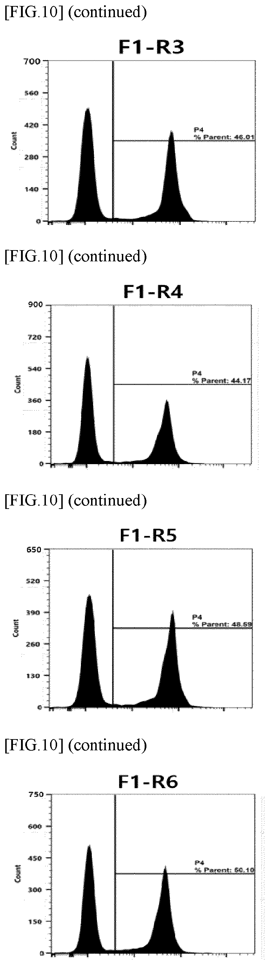

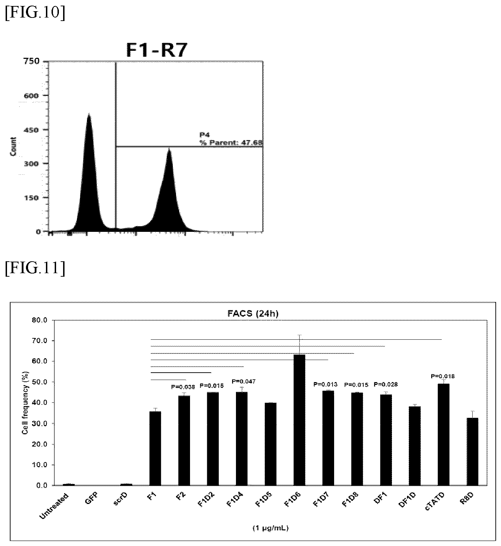

In addition, in the case of peptides (SEQ ID NOS: 16 to 21) in which an intermediate amino acid sequence of the F1 peptide is substituted with another amino acid (R), as shown in FIGS. 9 and 10, both peptide treated groups showed similar positive cell frequencies, and specifically, while showing positive cell frequencies of about 20% at 4 hours, about 40% at 24 hours, about 45% at 48 hours, and about 50% at 72 hours, the fluorescence expression increased over time. From the above results, it was determined that even when an intermediate amino acid of the F1 peptide is substituted with another amino acid (R), the cell permeability is maintained.

### 2-2. Analysis on cell permeability of peptides with SEQ ID NOS: 22 to 30 via flow cytometry

To determine whether the F1 peptide enables penetration into cells even if a peptide without cell permeability is linked in the middle of any amino acid sequence, splenocytes were isolated from ICR 8-week-old mice and cultured for 1 hour. Afterwards, 1µg/mL of fluorescent-labeled peptides (SEQ ID NOS: 22 to 30) was treated to cells respectively, followed by culture for 48 hours. Afterwards, flow cytometry was used to analyze the frequency of fluorescent-labeled cells over the culture time (24 and 48 hours) to determine the frequency of cells into which the peptide moved. Used as the control were peptides (cTATD and R8D) to which peptides (DDDDDDDD) that do not penetrate the plasma membrane were linked to cTAT and R8 peptides with cell permeability, respectively. The amino acid sequences of cTATD and R8D are as shown in Table 2 below. A total of 4 mice were used, with the number of mice per experimental group (Group) set to 2 and the experiment repeated twice.

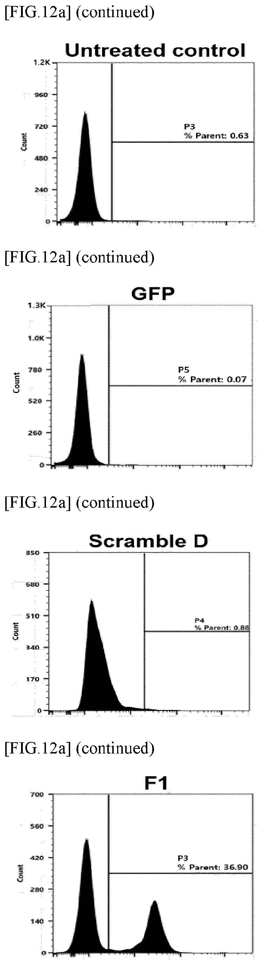

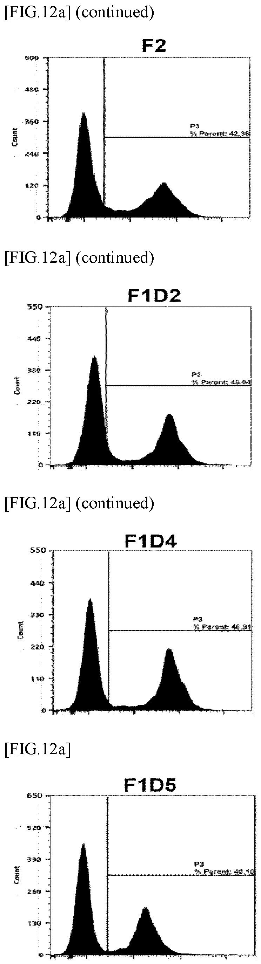

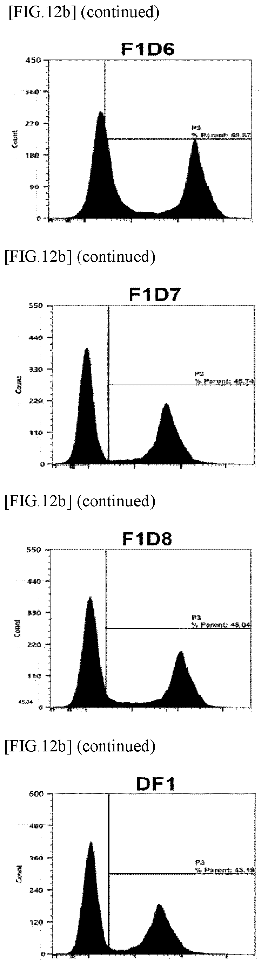

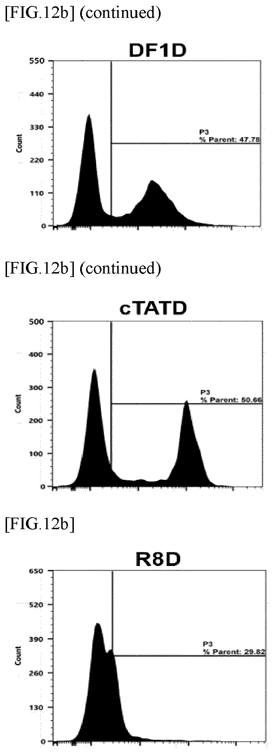

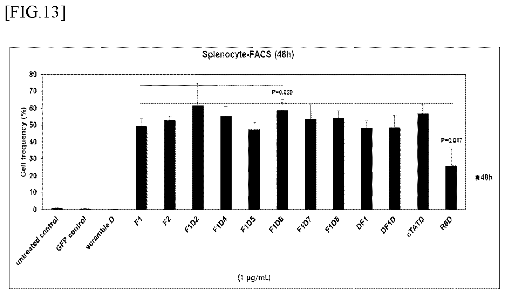

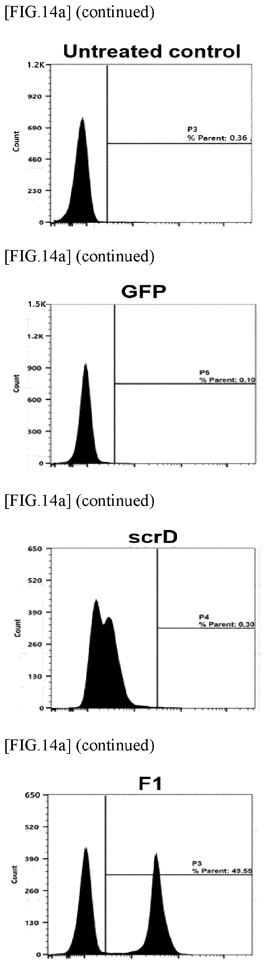

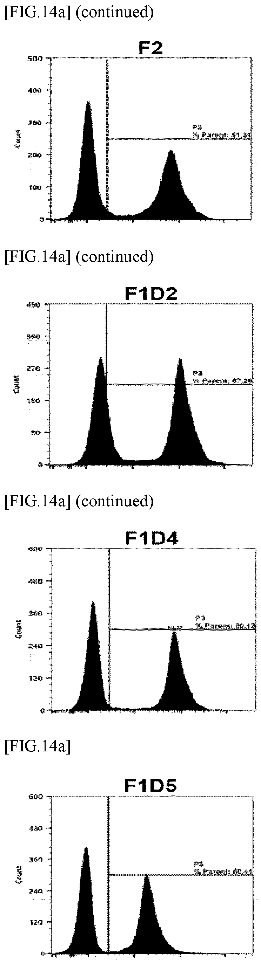

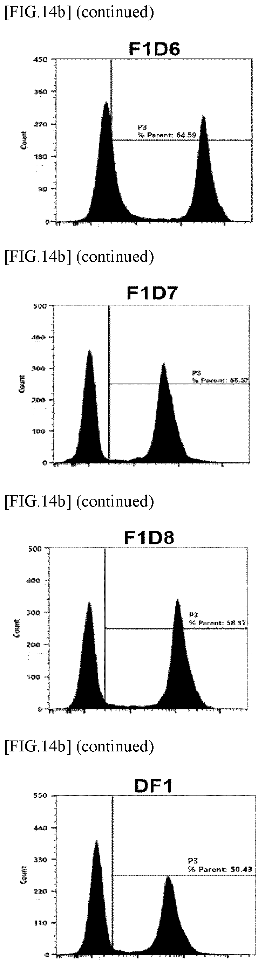

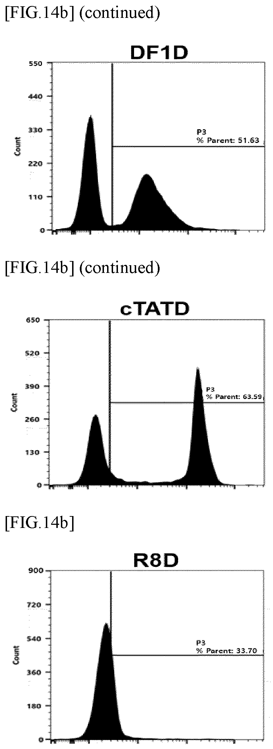

As a result, as shown in FIG. 11, FIGS. 12A-12B, FIG. 13, and FIGS. 14A-14B, when the F1D8 peptide was compared with the control cTATD and R8D peptides, it was found that the F1 peptide made the DDDDDDDD peptide penetrate inside the cell at a level similar to that of the cTAT peptide, and a degree of penetration for the DDDDDDDDDD peptide into the cell is superior to that of the R8 peptide. From the above results, it was found that the F1 peptide enables penetration into the cell even if the peptide without cell permeability is linked in the middle of any amino acid sequence.

**TABLE 2**

| Peptide | Amino Acid Sequence |
|---|---|
| cTATD | CYGRKKRRQRRRCDDDDDDDD |
| R8D | RRRRRRRRDDDDDDDD |

### 2-3. Analysis on cell permeability of peptides with SEQ ID NOS: 21 to 30 via confocal microscopy

In order to determine whether the F1 peptide may enable penetration into the cell even if the peptide without cell permeability is linked in the middle of any amino acid sequence, splenocytes were isolated from 8-week-old ICR mice and cultured for 1 hour. Afterwards, the cells were treated with 1 µg/mL of fluorescent-labeled peptides (SEQ ID NOS: 21 to 30), and culture was performed for 24 hours, followed by observation of intracellular fluorescence expression under confocal microscopy.

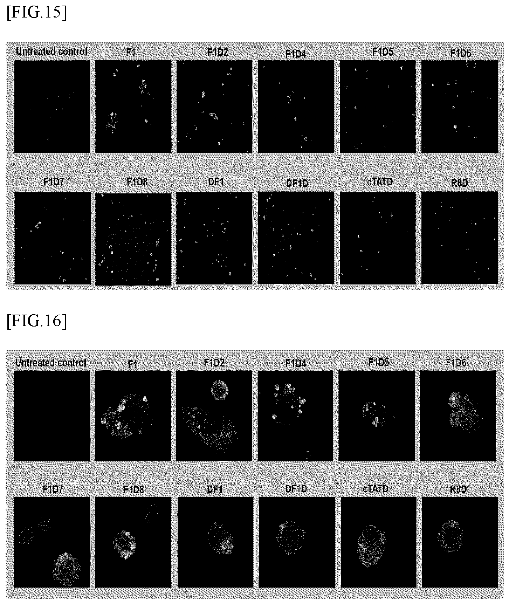

As a result, as shown in FIGS. 15 and 16, it was found that intracellular fluorescence expression was detected in both peptide treated groups with SEQ ID NOS: 21 to 30. From the above results, it was found that the F1 peptide may enable penetration into the cell even if the peptide without cell permeability is linked in the middle of any amino acid sequence.

### 2-4. Analysis on skin tissue infiltrability of peptides with SEQ ID NOS: 1 to 30 by confocal microscopy

In order to identify the skin tissue infiltrability of peptides with SEQ ID NOS: 1 to 30, the left leg of 4-week-old ICR mice was left unscratched, and the right leg was scratched with a needle. Afterwards, 1 µg/100 µL of peptides (SEQ ID NOS: 1 to 30) was applied to both legs with a cotton swab, after 4 hours, the tissue was collected and cryosectioned, and fluorescence expression in the tissue was observed by confocal microscopy.

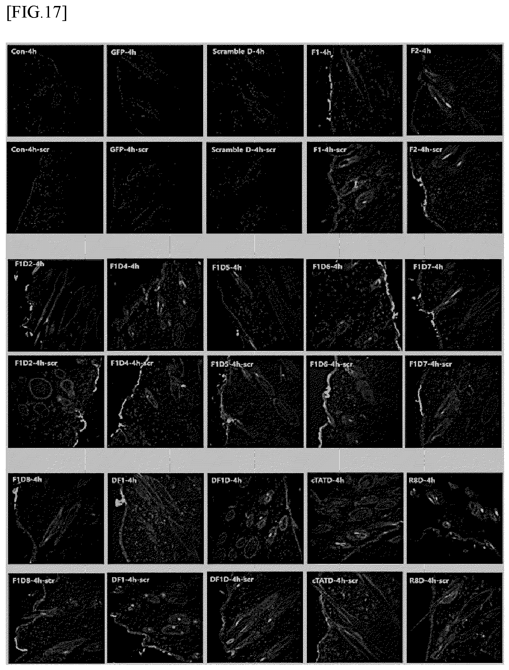

As a result, as shown in FIG. 17, fluorescence expression in tissues was detected in both groups treated with peptides with SEQ ID NOS: 1 to 30, and it was found that no significant difference was observed in the fluorescence expression with or without scratch. From the above results, it was found that even if an intermediate amino acid of the F1 peptide was substituted with another amino acid (R), the cell (tissue) permeability was maintained, and the F1 peptide enabled penetration into cells (tissues) even if the peptide without cell permeability was linked in the middle of any amino acid sequence.

Having described in detail specific parts of the content of the present disclosure above, it is clear for those of skilled in the art that these specific descriptions are merely preferred example embodiments, and the scope of the present disclosure is not limited thereby. In other words, the substantial scope of the present disclosure is defined by the appended claims and their equivalents.

## Claims

1. A peptide represented by the following general sequence formula:
[General Formula] X1-X2-X3-X4-X5-X6-X7
wherein the X1 is one selected from the group consisting of glycine, alanine, isoleucine, leucine, valine, asparagine, glutamine, arginine, lysine, aspartic acid, glutamic acid, tyrosine, tryptophan, phenylalanine, and proline,
the X2 is cysteine or arginine;
the X3 is cysteine or arginine;
the X4 is glycine or arginine;
the X5 is glutamic acid or arginine;
the X6 is isoleucine or arginine; and
the X7 is valine or arginine.

2. The peptide of claim 1, wherein the peptide consists of any one amino acid sequence of SEQ ID NOS: 1 to 21.

3. A peptide which comprises a peptide without cell permeability at N-terminus or C-terminus, or between each amino acid of the peptide according to claim 1.

4. A nucleotide having a base sequence encoding the peptide according to any one of claims 1 to 3.

5. A vector comprising the nucleotide according to claim 4.

6. A transformant transformed by the vector according to claim 5.

7. A composition for drug delivery, comprising the peptide according to any one of claims 1 to 3 and a desired drug.

8. The composition of claim 7, wherein the drug is a compound, protein, peptide, or nucleic acid.

9. The composition of claim 7, wherein, in the composition, the peptide and the drug form a covalent or non-covalent conjugate.

10. The composition of claim 7, wherein, in the composition, the peptide and the drug are covalently bonded to each other to form a conjugate.

11. The composition of claim 7, wherein the peptide is labeled with one selected from the group consisting of chromogenic enzymes, radioisotopes, chromophores, luminescent materials, fluorescers, super paramagnetic particles, and ultrasuper paramagnetic particles.
